# EUROPÄISCHE PATENTSCHRIFT

(11) **EP 0 878 173 B1**
(45) Veröffentlichungstag und Bekanntmachung des Hinweises auf die Patenterteilung: **09.08.2000**
(21) Anmeldenummer: 97121036.4
(22) Anmeldetag: 29.11.1997
(51) Int. Cl.: A61F 2/06

(54) **Stent-Graft**
Stent-graft
Stent-greffe

(30) Priorität: 14.05.1997 DE 19720115
(43) Veröffentlichungstag der Anmeldung: 18.11.1998
(73) Patentinhaber: Jomed Implantate GmbH, 72414 Rangendingen (DE)
(72) Erfinder: von Oepen, Randolf, Dr.-Ing., 72145 Hirrlingen (DE); Reifart, Nikolaus, Prof., 65817 Eppstein (DE)
(74) Vertreter: Schmitz, Hans-Werner, Dipl.-Ing.

(56) Entgegenhaltungen:
- WO-A-93/21860
- WO-A-95/24873
- WO-A-96/33672
- WO-A-97/24081

## Beschreibung

Die Erfindung betrifft einen Stent-Graft, wie er zur Unterstützung von Gefäßen, insbesondere bei Aneurysmen aber auch bei labilen bzw. brüchigen oder thrombotischen Gefäßwänden eingesetzt wird. Herkömmliche Stent-Grafts bestehen in der Regel aus einem radial aufweitbaren Stent, der beispielsweise durch Laserschneiden aus einem Metallröhrchen gefertigt wird, und einem daran angenähten Überzug aus einem Gewebe oder einer Folie. Der Überzug hat die Aufgabe, einen Blutdurchtritt bzw. einen Durchtritt von Blutbestandteilen oder Ablagerungen durch die Wandung des Stent-Grafts sowie ein Einwachsen von Gewebe durch die Wandung ins Innere des Stent-Grafts zu verhindern. Hierdurch wird unter anderem sichergestellt, daß die Gefäßwand vom Blutdruck entlastet ist und an der Implantationsstelle des Stent-Grafts keine Embolien auftreten können. Bei den herkömmlichen Stents entstehen aufgrund des Festnähens des Überzugs an dem Stent jedoch Knoten, die zu Verwirbelungen im offenen Durchfluß des Gefäßes mit der Gefahr einer Thrombenbildung führen. Besteht der Überzug aus einem Gestrick oder Geflecht, muß dieses um den Stent gefaltet werden. Ein faltenfreies Aufspannen des Überzugs beim Dilatieren des Stents ist dabei nicht immer gewährleistet.

Die WO 96/33672 erwähnt einen radial aufweitbaren Stent-Graft, der zwei aufweitbare Stents, zwischen denen eine röhrenförmige, flexible und dehnbare Materialschicht angeordnet ist, aufweist, wobei die Materialschicht über die Endbereiche des äußeren Stents nach außen umgeschlagen ist. Nachteilig hierbei ist, dass es beim Einführen dieses Stent-Grafts zu einem gegenseitigen Verrutschen der Stents kommen kann. Außerden ist die Herstellung der röhrenförmigen mittleren Materialschicht relativ aufwendig.

Die WO 97/24081 beschreibt eine Gefäßprothese, die sich aus zwei koaxial ineinander angeordneten wendelförmigen Trägern zusammensetzt, zwischen denen ein Gewebe angeordnet sein kann. Diese Vorrichtung ist jedoch zum Zusammenfalten für das Einbringen in ein Gefäß ungeeignet und kann sich nach dem Einbringen auch nicht selbständig dilatieren.

Der vorliegenden Erfindung liegt die Aufgabe zugrunde, einen Stent-Graft vorzuschlagen, der einfach in der Herstellung ist und problemlos in ein Gefäß eingeführt werden kann.

Zur Lösung dieser Aufgabe schlägt die Erfindung einen Stent-Graft mit den Merkmalen des Anspruchs 1 vor. Beim erfindungsgemäßen Stent-Graft kann ein Annähen der Materialschicht an einem der Stents entfallen, da die Materialschicht zwischen die beiden koaxialen Stents eingeklemmt wird. Darüber hinaus ist die Materialschicht beim Einführen des Stents in das Gefäß gegen Verletzungen geschützt. Aufgrund der flexiblen und dehnbaren Eigenschaften der Materialschicht kann diese gemeinsam mit den beiden Stents an der Implantationsstelle radial aufgeweitet werden. Um beim Einschieben ein gegenseitiges Verschieben der beiden Stents zu verhindern, sind die beiden Stents in ihren Endbereichen punktuell miteinander verbunden. Hierzu sind verschiedene Techniken einsetzbar. So können die beiden Stents beispielsweise miteinander verschweißt, verpreßt oder verklebt werden. Die Materialschicht aus einem körperverträglichen Material oder aus einem biologischen Gewebe, ist aus einem um den inneren Stent gewickelten Folien- oder Gewebeband hergestellt. Hierdurch ist eine besonders rationelle Fertigung des Stent-Grafts möglich. Als Materialien für die Materialzwischenschicht kommen beispielsweise Polytetrafluorethylen, Polyethylenterephthalat oder biologische Materialien wie autologe oder homologe Venen oder Arterien in Frage. Vorgedehntes Polytetrafluorethylen weist hierbei besondere Vorteile auf, da dieses Material eine Fibrillenstruktur besitzt, wodurch das Einwachsen des Stent-Grafts in die Gefäßwand begünstigt wird. Hierdurch entsteht mit der Zeit ein fester Verbund zwischen Gefäß und Stent-Graft.

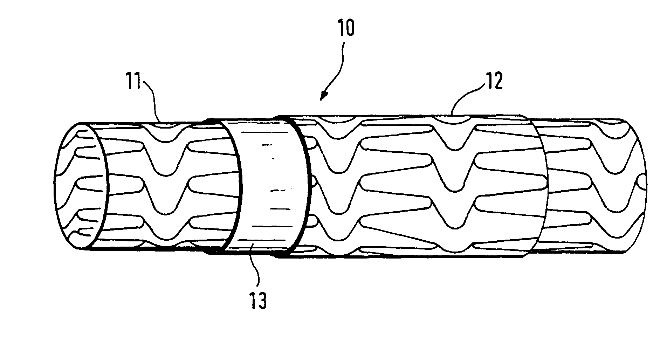

Nachfolgend wird ein bevorzugtes Ausführungsbeispiel eines erfindungsgemäßen Stent-Grafts anhand der Zeichnung näher beschrieben.

Die einzige Figur zeigt eine teilweise aufgeschnittene perspektivische Ansicht eines Stent-Grafts 10 mit zwei koaxialen Stents 11 und 12, zwischen denen eine dehnbare Materialschicht 13 angeordnet ist. Diese Schicht 13 kann aus einer körperverträglichen Kunststoff-Folie oder einem Kunststoffgewebe gefertigt sein. Aber auch die Verwendung anderer körperverträglicher oder biologischer Materialien ist möglich. Im dargestellten Beispiel erstreckt sich die Materialschicht 13 nur über einen Teil der Länge des Stent-Grafts 10.

## Patentansprüche

1. Stent-Graft mit zwei koaxial angeordneten und radial aufweitbaren Stents, wobei zwischen den beiden Stents (11,12) eine flexible, dehnbare Materialschicht (13) angeordnet ist, dadurch gekennzeichnet, daß die Stents (11,12) an ihren beiden jeweiligen Endbereichen punktuell miteinander verbunden sind, und daß die Materialschicht (13) von einem um den inneren Stent (11) gewickelten Folien- oder Gewebeband gebildet ist.

2. Stent-Graft nach Anspruch 1, dadurch gekennzeichnet, daß die Materialschicht (13) nur einen Teil der Stents (11, 12) überdeckt.

3. Stent-Graft nach einem der Ansprüche 1 bis 2, dadurch gekennzeichnet, daß die Materialschicht (13) von einem schlauchförmigen Element aus einer Folie oder einem Gewebe gebildet ist.

4. Stent-Graft nach einem der Ansprüche 1 bis 3, dadurch gekennzeichnet, daß die Materialschicht (13) aus Polytetrafluorethylen besteht.

5. Stent-Graft nach einem der Ansprüche 1 bis 3, dadurch gekennzeichnet, daß die Materialschicht (13) aus Polyethylenterephthalat besteht.

6. Stent-Graft nach einem der Ansprüche 1 bis 3, dadurch gekennzeichnet, daß die Materialschicht (13) aus einem biologischen Material besteht.

7. Stent-Graft nach Anspruch 6, dadurch gekennzeichnet, daß das biologische Material autologen Ursprungs ist.

8. Stent-Graft nach Anspruch 6, dadurch gekennzeichnet, daß das biologische Material homologen Ursprungs ist.

## Claims

1. A stent graft comprising two coaxially arranged, radially extendable stents, and a flexible, stretchable material layer (13) being arranged between said stents (11, 12), being characterized in that said stents (11, 12) are punctually connected with one another in their end regions, and that said material layer (13) is formed as a foil or fabric band which is wound around the inner stent (11).

2. Stent graft according to claim 1, being characterized in that said material layer (13) covers only a part of said stents (11, 12).

3. Stent graft according to claim 1 or 2, being characterized in that said material layer (13) is formed as a hose-shaped element composed of a foil or a fabric.

4. Stent graft according to one of claims 1 to 3, being characterized in that said material layer (13) is composed of polythetrafluorethylene.

5. Stent graft according to one of claims 1 to 3 being characterized in that said material layer (13) is composed of polyethylenteraphtalate.

6. Stent graft according to one of claims 1 to 3, being characterized in that said material layer (13) is composed of a biological material.

7. Stent graft according to claim 6, being characterized in that said biological material is an autologous.

8. Stent graft according to claim 7, being characterized in that said biological material is a homologous material.

## Revendications

1. Dilatateur-greffon avec deux dilatateurs coaxiaux et pouvant être élargis radialement, une couche de matériau (13) flexible et extensible étant disposée entre les deux dilatateurs (11, 12), caractérisé en ce que les dilatateurs (11, 12) sont reliés entre eux de façon ponctuelle sur leurs deux zones d'extrémité respectives, et en ce que la couche de matériau (13) est formée par une bande de feuille ou une bande de tissu enroulée autour du dilatateur (11) intérieur.

2. Dilatateur-greffon selon la revendication 1, caractérisé en ce que la couche de matériau (13) ne recouvre qu'une partie des dilatateurs (11, 12).

3. Dilatateur-greffon selon l'une quelconque des revendications 1 à 2, caractérisé en ce que la couche de matériau (13) est formée par un élément tubulaire à base d'une feuille ou d'un tissu.

4. Dilatateur-greffon selon l'une quelconque des revendications 1 à 3, caractérisé en ce que la couche de matériau (13) est à base de polytétrafluoroéthylène.

5. Dilatateur-greffon selon l'une quelconque des revendications 1 à 3, caractérisé en ce que la couche de matériau (13) est à base de polyéthylène-téréphtalate.

6. Dilatateur-greffon selon l'une quelconque des revendications 1 à 3, caractérisé en ce que la couche de matériau (13) est à base d'un matériau biologique.

7. Dilatateur-greffon selon la revendication 6, caractérisé en ce que le matériau biologique est d'origine autologue.

8. Dilatateur-greffon selon la revendication 6, caractérisé en ce que le matériau biologique est d'origine homologue.
